# EUROPEAN PATENT SPECIFICATION

(11) **EP 3 072 669 B1**
(45) Date of publication and mention of the grant of the patent: **08.05.2019**
(21) Application number: 15000842.3
(22) Date of filing: 23.03.2015
(51) Int. Cl.: B29C 65/48, B29C 65/00, B32B 38/00, B29C 59/14, A61F 13/511, A61F 13/15, B29C 65/52, B29L 31/48

(54) **APPLICATION OF ATMOSPHERIC PRESSURE PLASMA FOR IMPROVING ADHESION CAPACITY OF DISPOSABLE ABSORBENT ARTICLE COMPONENTS**
ANWENDUNG DER ATMOSPHÄRENDRUCKPLASMA ZUR VERBESSERUNG DER HAFTUNGKAPAZITÄT VON KOMPONENTEN VON ABSORBIERENDEN WEGWERFARTIKELN
APPLICATION DE PLASMA À PRESSION ATMOSPHÉRIQUE POUR AMÉLIORER L'ADHÉRENCE DE COMPOSANTS D'ARTICLES ABSORBANTS JETABLES

(43) Date of publication of application: 28.09.2016
(73) Proprietor: HAYAT KIMYA SANAYI ANONIM SIRKETI, 41275 Kocaeli (TR)
(72) Inventor: Akaytay, Arif, 41275 Basiskele/Kocaeli (TR); Acar, Haluk Ozgur, 41275 Basiskele/Kocaeli (TR); Askin, Sule, 41275 Basiskele/Kocaeli (TR)

(56) References cited:
- EP-A1- 2 489 511
- WOLF R A ET AL: "MODIFYING SURFACE FEATURES - Optimising extrusion coating/lamination seal strength by surface treatment", POLYMERS, LAMINATIONS, ADHESIVES, COATINGS, EXTRUSIONS : TAPPI PLACE CONFERENCE 2007 ; SEPTEMBER 16 - 20, 2007, ST. LOUIS, MISSOURI, USA, CURRAN ASSOCIATES, INC, RED HOOK, NY , vol. 2 17 September 2007 (2007-09-17), pages 881-904, XP002758042, ISBN: 978-1-60560-062-8 Retrieved from the Internet: URL:http://www.tappi.org/content/events/07 place/papers/wolf1.pdf

## Description

### Technical Field

The present invention is related to a method of improving adhesion capacity of a synthetic polymer nonwoven surface to a synthetic polymer nonwoven surface or to a synthetic polymer film surface.

### Background Art

The adhesion of synthetic polymer nonwoven and polymer film surfaces or polypropylene nonwoven and polypropylene nonwoven surfaces to each other has already been achieved by laminating these two surfaces with an adhesive or glue.

The processes of pretreatment of polyolefin materials to decrease their surface tension for providing adhesive action efficacy are known in the art. The corona treatment is the simple plasma treatment method which is used for this purpose is generated by the application of high voltage to sharp electrode tips which forms plasma at the ends of the sharp tips. On the other hand, corona treatment requires high frequency power generator, a high voltage energy and provides a two dimensional treatment which means weak bonding capability.

In view of the above problems, for the treatment of synthetic polymer nonwoven materials in the above mentioned object vacuum plasma treatment seems to be a promising method in the art.

EP0127149 describes a vacuum plasma treatment process for thermoplastic resins that the treatment temperatures are at least 5 °C higher than their melting or softening point and requires a closed chamber.

EP0436918 is related to low pressure plasma treatment for polyolefin films. The film is carried past the electrode at a speed of 30 meters/minutes with 0.6 seconds exposure times.

On the other hand, similar activation of surface can be obtained by cold plasma process as presented in patent application WO2004113426. The improvements of adhesion capacity of fibres are provided by processing them inside a reactor through exposure to cold plasma. The fabric travel speed is 15 meters/minutes for polyamide film with 30 seconds exposure times and 5 meters/minutes for polyethylene film with 80 second exposure times.

US4900388 also requires a cold plasma reactor for lamination of two polymeric sheets.

Each of these methods have disadvantages such as requirement of high voltages and a vacuum reactor for processing plasma treatment which is not allowing a selectively treatment of the surfaces and not appropriate to use them at a high speed production lines. Considering these disadvantages, it can be seen that all plasma treatment methods are not appropriate for all kind of polymers based on their application area EP 2 489 511 A1 discloses a method for laminating a polyolefin film to a synthetic polymer film while moving on rollers without using adhesives, comprising activating the surface of a film by atmospheric plasma treatment. Wolf et al., "MODIFYING SURFACE FEATURES - Optimising extrusion coating/lamination seal strength by surface treatment", POLYMERS, LAMINATIONS, ADHESIVES, COATINGS, EXTRUSIONS : TAPPI PLACE CONFERENCE 2007 ; SEPTEMBER 16 - 20, 2007, ST. LOUIS, MISSOURI, USA, CURRAN ASSOCIATES, INC, RED HOOK, NY,vol. 2 17 September 2007, pages 881-904 discloses a method for laminating two synthetic-polymer films (OPP and PET) while moving at 90 m/min by treating both with atmospheric plasma and joining them via an adhesive which is an extruded LDPE layer.

In despite of the fact that the plasma treatment improves the adhesion capacity of the polymers, it is not preferred for high speed production lines because plasma can not be applied continuously on a smooth surface before the application of an adhesive material. The inventor found out that discontinuous plasma pretreatment is enough to improve of bonding strength of nonwoven materials based on its porous surface structure. The dispersion of adhesive material on the plasma treated porous nonwoven surface provides an important improvement of bonding strength between polypropylene nonwoven surface and polymer film or polypropylene nonwoven surfaces. It is an object of the present invention to provide an improved process for the adhesion of polypropylene nonwoven surface to polymer film or polypropylene nonwoven surface to polypropylene nonwoven surface to use less adhesive material by pretreating the polypropylene nonwoven surface with atmospheric pressure plasma which additionally gives less damages to the material and the application speed of it is appropriate for high speed production lines.

It is an object of the present invention to provide an improved process for the adhesion of polypropylene nonwoven surface to polymer film or polypropylene nonwoven surface to polypropylene nonwoven surface to use less adhesive material by pretreating the polypropylene nonwoven surface with atmospheric pressure plasma which additionally gives less damages to the material and the application speed of it is appropriate for high speed production lines.

### Summary of invention

The invention is defined in claim 1. Further embodiments are defined in claims 2-13.

It is an aim of the present invention to provide a method to improve adhesion capacity between a synthetic polymer nonwoven and synthetic polymer nonwoven or synthetic polymer film by preatreating the synthetic polymer nonwoven surface with atmospheric pressure plasma. It is also an aim of the present invention to use this method to adhere a base sheet material and hook material for the production of fastening tapes by using less adhesive material. It is an another aim of the present invention to use this method to adhere fastening tapes to the side panels of absorbent articles by using less amount of adhesive material.

The present invention provides an appropriate pretreatment method for high speed production lines at low temperatures to improve adhesion capacity and acceptable delamination force by using less adhesive material and gives less damages to the materials such as polypropylene nonwoven.

In the present invention the range of temperature for the application of atmospheric pressure plasma is between 160-240 °C and the contact temperature with the polymer nonwoven surface is between 3-25 °C.

In one aspect of the present invention nitrogen is used to obtain plasma.

In another aspects of the present invention, air, oxygen, hydrogen, argon, helium, xenon, carbon dioxide, nitrous oxide, nitrogen monoxide and nitrogen dioxide or a mixture of two kinds or more is used to obtain plasma.

### Brief description of drawings

Fig. 1 is a plan view of a fastening tape according to the present invention.
Fig. 2 illustrates the atmospheric pressure plasma treated regions of a fastening tape.
Fig. 3 is a plan view of fastening tapes manufactured by the process illustrated in Fig. 7.
Fig.4 illustrates the atmospheric pressure plasma treated regions of a fastening tapes manufactured by the process illustrated in Fig. 7.
Fig. 5 illustrates a disposable absorbent article comprising fastening tapes according to the present invention.
Fig. 6 illustrates a sanitary napkin comprising plasma treated regions according to the present invention.
Fig. 7. schematically illustrates an embodiment of the process of the present invention.

### Detailed description of the present invention

It is often necessary to bond a polymer material to an another polymer material for various kind of applications. In order to achieve that wetting of the surface of the material with an adhesive or glue is necessary. Polymer materials cannot be wetted with an adhesive directly without lowering its surface tension by a pretreatment method. Polyethylene and polypropylene for example have very low surface energy. Pretreatment of these and other engineered plastics improves bonding prior to printing, coating, and laminating. Various techniques are known in the art for pretreatment of these kinds of surface layers.

The known processes, more particularly the process comprising a plasma treatment, are frequently employed for the adhesion of polymer films, sheets or foils to, polymer films, sheets, foils or other substrates. The plasma is described in more detail in the literature which is a matter that exists in the form of ions and electrons. Basically, it is a gas that's been charged electrically with freely moving electrons in both the negative and positive state. Since plasma consists of electrons, molecules or neutral gas atoms, positive ions, UV light along with excited gas molecules and atoms, it carries a good amount of internal energy. Surfaces in contact with the gas plasma are bombarded by these energetic species and their energy is transferred from the plasma to the solid. These energy transfers are dissipated within the solid by a variety of chemical and physical processes to result in a unique type of surface modification that reacts with surfaces in depths from several hundred angstroms to 10 µm without changing the bulk properties of the material which can be called as a three dimensional surface modification.

The physical characteristics of plasma can be affected from a wide variety of parameters in a high ratio and subsequently these affect the surface chemistry obtained by plasma modification. Processing parameters, such as gas types, treatment power, treatment time and operating pressure, can be varied by the user. However system parameters, such as electrode location, reactor design, gas inlets and vacuum are set by the design of the plasma equipment. This broad range of parameters offers greater control over the plasma process than that offered by most high-energy radiation processes.

Plasma treatment, on the other hand, as a dry and eco-friendly technology, is offering an attractive alternative to add new functionalities such as water repellence, long-term hydrophilicity, adhesion strength, mechanical, electrical and antibacterial properties as well as biocompatibility due to the nano-scaled modification on textiles and fibers. At the same time, the bulk properties as well as the touch of the textiles remain unaffected.

Plasma processes can be grouped into two main classes as low density and high density, according to their electron temperature versus electron density. There are different surface modification application techniques with plasma which are based on these classes such as; low pressure and atmospheric plasma. However, low pressure plasma is generated in closed vacuum chamber, for that reason it does not have inline production capability and also, a bigger pump is required to reach the necessary process pressure. The cold plasma method also requires a vacuum reactor, high temperature and high energy levels for the application. Due to the requirement of a vacuum chamber, they are not appropriate for inline production adaptation and also have high energy and temperature requirement. As such its use is restricted and disadvantageous for the treatment of polymer nonwoven surfaces inline.

Atmospheric pressure plasma as the choice of the present inventors has an important advantage such as no reaction vessel is needed to ensure the maintenance of a pressure level thereby it can be integrated in the production line which is the aim of the present inventor. Adhesion capacity of a polymer nonwoven can be improved by implementing atmospheric pressure plasma method to provide ease of handling and cost saving.

The synthetic polymer nonwoven fabrics are widely used in disposable articles, such as diapers, feminine care products, wipes, exc. and also, battery and electrolytic capacitor separators, filtration media, substrates for battery electrodes, and the like thanks to its beneficial properties and low cost ratios. They can be laminated with an another synthetic polymer nonwoven or synthetic polymer film based on their application area.

The main problems of laminated synthetic polymer materials used as a part of fastening system for absorbent articles are low bonding strength between layers and peel-off behaviour of the synthetic polymer nonwoven fabrics which are commonly used in laminated materials. However, surface pretreatment might be beneficial to improve their adhesion to each other before adhesion of the layers with an adhesive material.

It should be also pointed out that all plasma methods may not be suitable for all kind of polymers based on the aim of the application. There are few studies in the literature studying the effect of plasma treatment on the peel-off behaviour of laminated textile fabrics. However, surprisingly, atmospheric pressure plasma method has not been applied on synthetic polymer nonwoven fabric to improve adhesion strength of it since it is bonded with synthetic polymer film or polymer nonwoven by lowering the amount of adhesive material. The adhesion of synthetic polymer nonwoven materials to a synthetic polymer nonwoven or polymer film have a great importance in hygienic textile industry especially for the production of absorbent articles.

Various kinds of adhesive materials can be used for the lamination of synthetic polymer materials. However, the amount of adhesive material which is used to laminate the layers of a fastening system affects the thickness of the material and also according to their high cost level, decreasing of their consumption which is important for industrial scale.

A typical fastening system for use with absorbent articles may have a fastening tape and a landing member (also known as a landing zone). The fastening tape may be disposed directly or indirectly upon the longitudinal edge of the body portion in either the front or back of the absorbent article waist regions. In use, the fastening tape may be secured to the landing member, which is disposed upon the correspondingly opposite body portion of the front or back of the absorbent article. A refastenable system may be provided with, e.g., hooks on the fastening tape that releasably engage loops disposed on the landing member, or vice versa. To improve fit, the end of the fastening tape that does not engage the loops in the landing member area, may be attached to one end of an elastic/stretch member (ear), and the other end of the elastic/stretch member may be secured to the longitudinal edge of the body portion of the absorbent article.

A fastening tape for an absorbent article has a base sheet configured by a nonwoven fabric material and a hook sheet in which a plurality of engagement hooks are provided.

As used herein, the term "absorbent article" refers to devices which absorb and contain body exudates, and, more specifically, refers to devices which are placed against or in proximity to the body of the wearer to absorb and contain the various exudates discharged from the body.

As used herein, the term "synthetic polymer nonwoven material" is used in its normal sense and specifically, refers to a sheet like structure (e.g. web) that has a structure of individual fibers or threads which are interlaid, but not in any regular, repeating manner. A nonwoven material can be a single layer structure or a multiple layer structure. Each layer in a nonwoven material can include one kind of fibers or two or more kinds of fibers, with each kind of fiber configured in any way described herein or known in the art. A nonwoven material can also be joined to another material, such as a film, to form a laminate. A nonwoven web can be bonded to provide integrity to the web and/or to attach the nonwoven web to another material.

A nonwoven material can be made from various natural and/or synthetic materials. Exemplary natural materials include cellulosic fibers, such as cotton, jute, pulp, and the like; and also include reprocessed cellulosic fibers like rayon or viscose. Notably, a nonwoven material can be made from renewable materials. Exemplary synthetic materials include but not limited to synthetic thermoplastic polymers that are known to form fibers, which include, but are not limited to, polyolefin (e.g.polyethylene, polypropylene, polybutylene and the like); polyamides (e.g. nylon 6, nylon 6/6, nylon 10, nylon 12 and the like); polyesters (e.g. polyethylene terephtlate, polybuthylene terephthalate, polylactic acid and the like); polycarbonate; polystyrene; thermoplastic elastomers; vinyl polymers; polyurethane; as well as blends and copolymers thereof, and any additives or processing aids known in the art. Any of these materials can be used to form one or more mono-component fibers, and any combination of any of these materials can be used to form one or more of any kind of multicomponent fibers in any configuration.

Fibers of a relatively short length (e.g. 40 mm or less) are typically manufactured into a nonwoven using processes like drylaying (e.g. carding or airlaying) or wetlaying. Continuous fibers or filaments can be spun out of molten thermoplastics or chemical solutions and formed into a web using spunlaying/spunbonding, meltblowing, or electrospinning by exempla. Another process for forming a nonwoven is film fibrillation. These processes can also be combined to form composite or layered fabric structures.

The term "hook material" as used herein refers to male fastening elements that include stems with or without loop-engaging heads that have an overhang.

As an example, a fibrous material used as a fastening material generates the fastening type by laminating with a hook material, can be a nonwoven material made of polypropylene (100%).

The basis weight of polymer nonwoven fastening member can be 10-100 grams per square meter, or any integer value for grams per square meters between 10 and 100, or any range formed by any of these values.

The hooks can be bonded or laminated with polymer nonwoven materials in various patterns and hook densities. For example, the hooks can be arranged in rows and/or columns, or any other arrangement of hooks known in the art. In various embodiments, the fastening type can have a hook density of 10 and 1000 hooks per square centimeter, or any integer number of hooks between 10 and 1000, or any range formed by any of these values.

The method of this invention is used for improving bonding strength between a synthetic polymer nonwoven and a synthetic polymer film or polymer nonwoven in high speed production lines by applying atmospheric plasma on the surface of synthetic polymer nonwoven material as a pretreatment method for lowering its surface tension before adhere them to each other.

Any method capable of attaching the first and second substrate to one another, and/or the synthetic polymer nonwoven or polymer nonwoven to the synthetic polymer nonwoven, is believed suitable for use in the present invention.

For example, the materials may be attached together by hot or cold melt adhesives, thermal bonding, sewing, combinations of these methods, and the like. In one preferred embodiment of the present invention the hook material is bonded to the atmospheric pressure plasma pretreated base sheet material with hot melt adhesive. In an another embodiment of the present invention atmospheric pressure plasma pretreated base sheet material is bonded to the side panel of an absorbent article with hot melt adhesive. By this way, the adhesive material can be adsorbed on the surface efficiently. A single jet atmospheric plasma system with product code PFW10 which has 16-25 kHz working frequency and 3-5 kV electrode volts with up to 900 m/min relative speed to surface is supplied from Plasmatreat Company. Since the production speed of a fastening system for an economically feasible production line is preferably between 50 and 250 meters/minutes (m/min), most preferably 225 m/min, atmospheric pressure plasma equipment having adjustable speed is the most convenient system for inline application for the mentioned object.

The most preferred embodiment of the invention is the method that said single jet plasma electrode is integrated in line by keeping preferably between 2-15 millimeters, most preferably 4 milllimeters distance between a jet front and the substrate material and the working frequency is preferred to be at least 16 kHz. Since the plasma is passed through from the electrode the temperature of it is preferably between 160-240 °C, most preferably around 200 °C. The contact temperature with the fabric is 0-50 °C, most preferably 3-25 °C which makes the atmospheric pressure plasma the most suitable method for the pretreatment of synthetic polymer nonwoven materials. Preferably air or nitrogen is used, most preferably nitrogen is used to obtain plasma based on its convenience to hygiene industry. It also gives less damage to the surface of the nonwoven materials. Air, oxygen, hydrogen, argon, helium, xenon, carbon dioxide, nitrous oxide, nitrogen monoxide and nitrogen dioxide or a mixture of two kinds or more may be used.

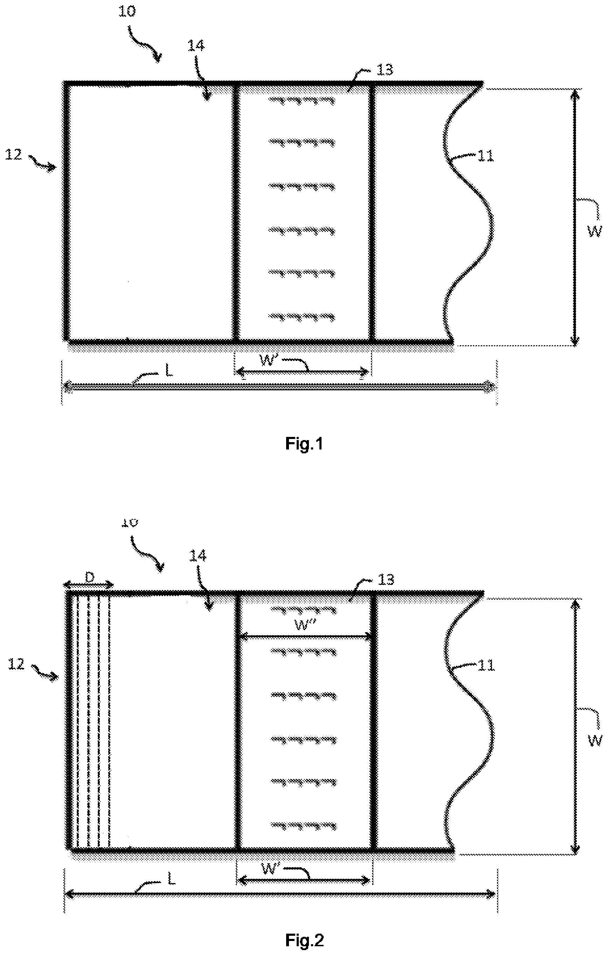

The fastening tape can be best understood by reference to the figures wherein Fig. 1 illustrates a plan view of a fastening tape according to one aspect of the present invention. The fastening tape 10 has a length L and a width W. As used herein, reference to the length of a fastening tape is intended to refer to the dimension of the fastening tape generally perpendicular to the length of the disposable absorbent article to which it is attached. The length of a disposable absorbent article refers to that dimension of the garment which extends from a rear waistband area of the garment, to an opposite waistband area of the garment. This generally corresponds to the machine direction of the absorbent article during manufacture and the greatest planar dimension of the product. The width of the fastening tape refers to the dimension of the fastening tape generally perpendicular to the length thereof.

The fastening tape 10 defines a first transverse edge 12 and a second transverse edge 11. The fastening tape 10 comprises a base sheet 14 having a width W. In the illustrated embodiment, the width W of the base sheet corresponds to the overall width of the fastening tape 10. A hook material 13 is attached to the base sheet 14 and extends the entire width W of the fastening of the fastening tape 10. The hook material is longitudinally spaced from both the first transverse edge 12 and the second transverse edge 11. The hook material has a width W' extending along the length direction of the fastening tape and a length perpendicular to the width W'.

An adhesive material is applied on base sheet material 14 for attachment of hook material 13 to the base sheet material 14 through the width of hook material W'.

Fastening tapes which is defined in present invention suitably have a width of from about 15 millimeters to about 100 millimeters, preferably of from about 25 millimeters to about 50 millimeters, and a length of from about 50 millimeters to 150 millimeters, preferably of from about 50 millimeters to 125 millimeters. The hook material may have a width (W') of from about 5 millimeters to about 50 millimeters, preferably of from 10 millimeters to about 30 millimeters. The atmospheric pressure plasma pretreatment may be applied on the base sheet material which have a width (W") of from 2.5 millimeters to 50 millimeters, preferably 5 millimeters to 30 millimeters which is longitudinally offset through the region where hook material 13 is adhered on with an adhesive material. Applicants have discovered that pretreatment of base sheet material 14 by atmospheric pressure plasma increased bonding strength between non atmospheric plasma pretreated hook material 13 and atmospheric plasma pretreated base sheet material 14.

Also, they discovered that pretreatment of base sheet material through its targeted region D which is shown in Fig.2 that is adhered to side flap of an absorbent article increased adhesion capacity between fastening tape 10 and side flap 24. The atmospheric pressure plasma treatment may be applied on base sheet material 14 along the length direction of the fastening tape and a length perpendicular to the width W' through the width of from first side edge up to 25 millimeters, preferably 0.5 millimeters to 15 millimeters.

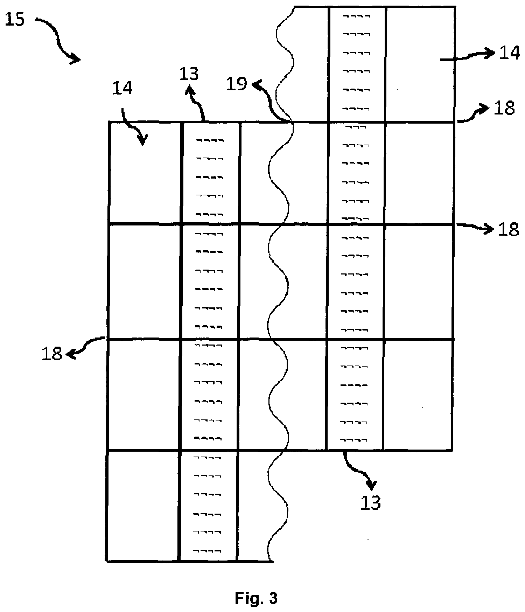

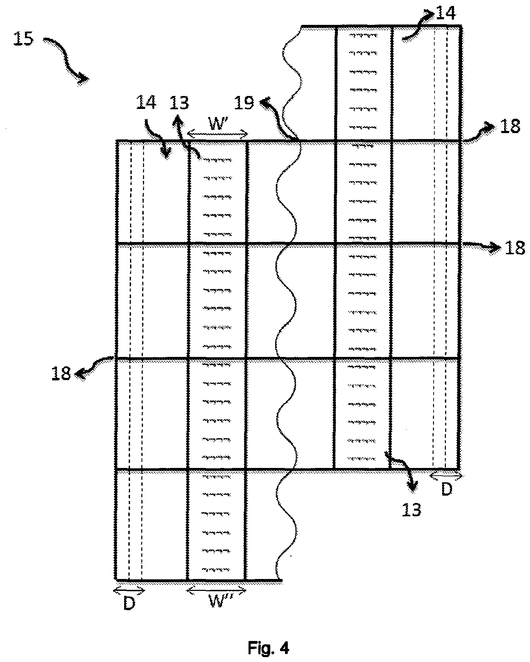

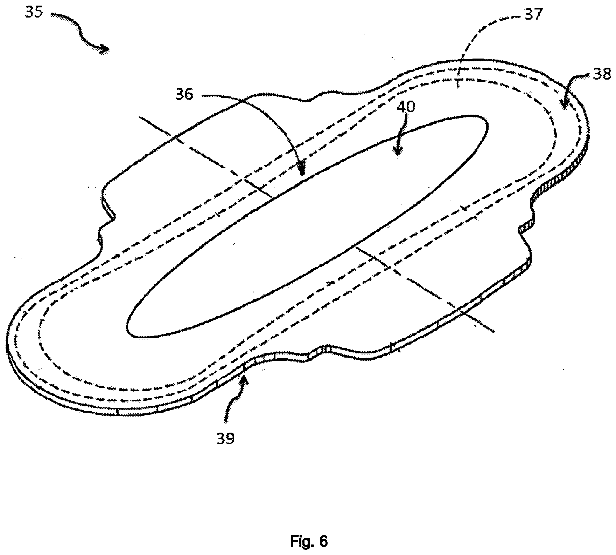

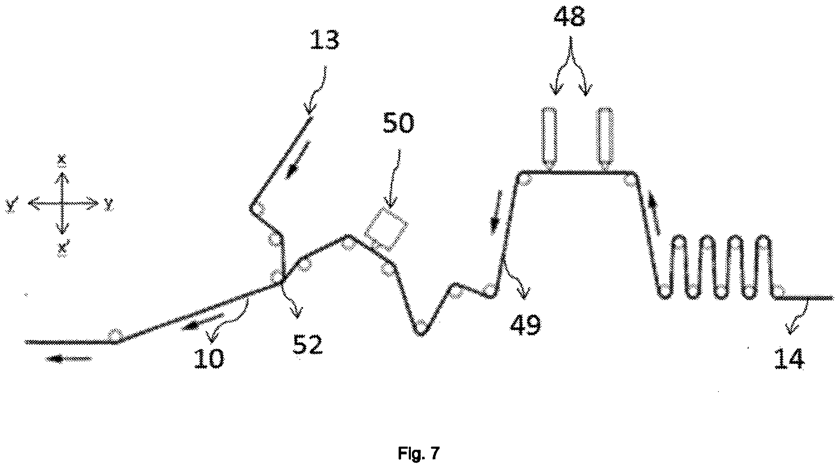

Fig. 3 and Fig. 4 illustrate the fastening tapes formed by the process illustrated in Fig.7. As can be seen from reference to Figs. 3 and 4, the fastening tapes produced by the method illustrated in Fig.7 comprise a base sheet material 14 and hook material 13 which are adhered to each other with an adhesive material. The adhesives used to form the fastening tapes 15 are not illustrated in Fig.1, 2, 3 and 4. Cut lines 19 are defined for the production line of an absorbent article and that process is not illustrated in Figures. The fastening tape 15 is produced without cutting process from cut line 19 as illustrated in Fig. 6. Atmospheric pressure plasma is longitudinally applied on base sheet material through width W". The individual fastening tapes can be attached to an absorbent article by adhering the base sheet material after cutting the fastening tapes 15 from their cut lines 18 and 19.

In an another aspect, Fig. 4 illustrates a fastening tape 15 having atmospheric plasma pretreated regions D and W".

In a preferred embodiment illustrated in Fig. 1 and Fig. 2 base sheet material 14 is longitudinally pretreated with atmospheric pressure plasma through the width W".

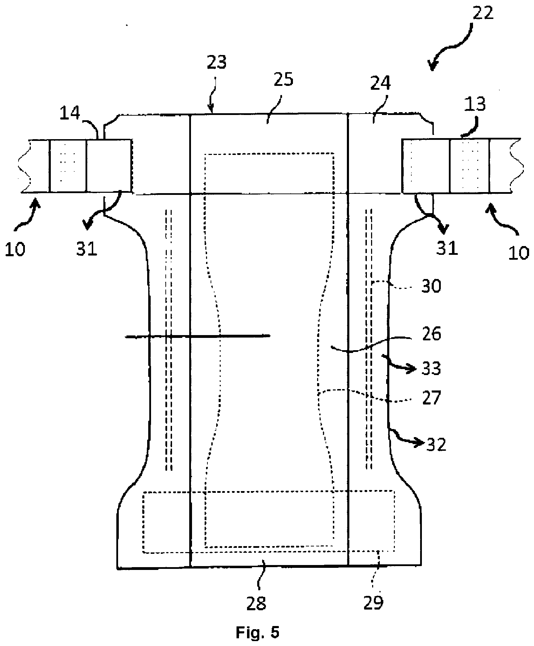

Fig. 5 illustrate a fastening tape according to the present invention in use on a disposable diaper. Those skilled in the art will recognize that diaper 22 generally comprises an outer cover 32, an absorbent main body 23, an absorbent core 27 which is located between the outer cover 32 and a topsheet 33. Leg elastic members 30 are located generally at the longitudinal edges of the diaper 22. The diaper further comprises a front waistline portion 28, crotch portion 26 and landing zone 29. The side panels 24 are generally elastic materials and attached at the rear side of the diaper by sandwiching the outer cover 32 and topsheet 33. The fastening tape 10 of the present invention is generally separated the two pieces of hook materials 13 to form two slit tape from their cut lines 18, 19 before attached on the side panels 24 at the rear part of the diaper. In the illustrated embodiment the portion of the fastening tapes according to the present invention is joined to the diaper 22. The attached portion of the fastening tapes on the diaper have a width of from 5 millimeters to 50 millimeters, preferably of from 10 millimeters to 30 millimeters. An adhesive material is applied through the defined portion of the fastening tape 10 which comprise the width D of the fastening tape where is pretreated with atmospheric pressure plasma by the method illustrated in Fig. 7.

Fig. 6 illustrates a sanitary napkin product 35 which is having atmospheric pressure plasma pretreated regions according to the present invention. The sanitary napkin 35 herein has a topsheet material 37, a backsheet material 39, an absorbent core 36 which takes place between topsheet 37 and backsheet 39 and the below the topsheet a liquid transfer layer 40 is positioned on the absorbent core 36. The topsheet 37 and backsheet 39 are joined to each other at the side edges 38 by any method and atmospheric pressure plasma is applied according to the present invention on the inner side of the topsheet material 37 at the regions where the topsheet 37 is joined to the backsheet 39.

Fig. 7 illustrates a schematic view of a process of manufacturing of fastening tape according to the present invention. In the embodiment illustrated in Fig.7 base sheet material 47 has a width W extending along in the direction y'. Atmospheric pressure plasma is applied on targeted regions of base sheet 14 by single jet plasma electrodes 48. In another aspect of the present invention the atmospheric pressure plasma is applied on additional region which has width D of base sheet material 48, (may be defined as hook material non-existent region) which is attaching on a side panel 24 of an absorbent article. Additional atmospheric pressure plasma single jet electrodes 48 to pretreat the base sheet material 14 may be used for this purpose. A hot melt glue is longitudinally applied through the width W' of the atmospheric pressure plasma treated base sheet material 49 by an adhesive applicator 50. The hook materials 13 are attached to the atmospheric pressure plasma treated regions of base sheet material 14, as a result of the adhesive applied to the base sheet material 14 by the adhesive applicator 50. The adhesion of hook material 13 and base sheet material 14 is provided by means of an infeed roller 52 with changing strain force. The attachment of the hook material 13 to the base sheet material 14 forms a laminate which is defined as fastening tape 10.

### Example 1

### Bonding of synthetic polymer nonwoven materials to synthetic polymer films in the hygiene industry as a fastening tape of a fastening system

In hygiene industry, mentioned bonded material was used as a fastening tape attachment component for a baby diaper. The production of the fastening tape material was performed at a lamination line continuously by lamination of synthetic polymer nonwoven and synthetic polymer film with hotmelt adhesive material. The synthetic polymer nonwoven material was polypropylene and the synthetic polymer film was a polyolefin based material which were bonded with hotmelt adhesive. The polypropylene nonwoven material was treated with single jet atmospheric pressure plasma equipment of Plasmatreat Company prior to adhesive application The polyolefin film may be treated with suitable plasma method. Hotmelt adhesive is applied on by an adhesive applicator and said materials are laminated to each other.

The polypropylene nonwoven fabric, with 65 grams/m² was subjected to plasma processing in the following conditions:

**Table 1**

| **Atmospheric Pressure Plasma Application Conditions** | |
|---|---|
| **Applied gas** | Nitrogen |
| **Pressure** | 2 bar |
| **The distance between nozzle and substrate** | 4 millimeters |
| **Electrode voltage** | 3-5 kV |
| **Contact temperature to the substrate** | 5-20 °C |

### Example 2

To compare the delamination strength between polymer nonwoven base sheet material and polyolefin hook material, atmospheric pressure plasma is applied to polymer nonwoven base sheet material in above conditions which is mentioned at Table 1. Applied hotmelt adhesive amounts were reduced in the range of 30% and 70% with regard to the amount of applied hotmelt adhesive of non plasma treated fastening tapes which is applied in 51.00 g/m² amount. The delamination strength between polypropylene nonwoven base sheet and polyolefin hook material were measured for plasma treated samples having different amount of adhesives (15.30, 25.50, 35.70 g/m²) and non plasma treated fastening tape having 51.00 g/m² hotmelt adhesive. The results are to be seen in Table 2. Atmospheric pressure plasma treatment is applied as explained in the Example 1.

### Comparative Example 1

### Bonding Process of non plasma treated fastening components

For comparison proposes in the example 1 mentioned process is applied except the fact that the atmospheric pressure plasma is not applied before adhesive application and the amount of applied adhesive is 51.00 g/m².

**Table 2**

| **Comparison of Delamination force of atmospheric pressure plasma treated nonwoven with varying adhesive amount and non plasma treated nonwoven** | | | | |
|---|---|---|---|---|
| | **Non atmospheric plasma treated PP NW laminated with corona treated polyolefin film** | **Atmospheric plasma treated PP NW laminated with corona treated polyolefin film** | | |
| **Amount of applied adhesive for lamination (grams/m²)** | 51.00 | 35.70 | 25.50 | 15.30 |
| **Delamination force (N)** | 19.45 | 20.03 | 18.29 | 18.01 |

As can be seen from reference to Table 2, pretreatment of nonwoven material with atmospheric plasma produces a fastening tape having generally acceptable delamination force by using 30%, 50% or 70% less amount of adhesive material.

### Example 3

### Bonding of synthetic polymer nonwoven material to synthetic polymer nonwoven material in the hygiene industry as bonding of a fastening tape to a side panel

The tensile strength test between laminated fastening tape to side panel of a diaper was made for two samples. One of the fastening tape sample has a base sheet material which is non plasma treated and adhered to side panel with 102.56 g/m² hotmelt adhesive material. The other fastening tape sample has a base sheet material which is pretreated with plasma through a part of region where it is adhered to a side panel with 51.28 g/m² hotmelt adhesive material. Base sheet material is comprising of 100% polypropylene nonwoven (PP NW), side panel material is comprising of 100% polypropylene nonwoven.

**Table 3**

| **Comparison of Tensile strength test results of atmospheric plasma treated nonwoven and non plasma treated nonwoven** | | | |
|---|---|---|---|
| **Test parameters** | **Amount of applied adhesive for lamination (grams/m²)** | **Tensile Strength** | |
| | | **Fₘₐₓ (N/33 mm)** | **E_{break} (%)** |
| **PP NW side panel laminated with fastening tape having non atmospheric plasma treated PP NW [Example 3]** | 102.56 | 56,30 | 159,48 |
| **PP NW side panel laminated with fastening tape having atmospheric plasma treated PP NW [Example 3]** | 51.28 | 52,57 | 143,05 |

### Test Methods

### Lamination Strength Test

The test chosen to determine the delamination force of the two polymer materials is the lamination strength test according to WSP (World Strategic Partners) 401.0. The test is performed by Zwick/Roell tensile machine with a speed of 300 mm/min without any load cell. The distance between the lower and the upper clamps is 25 mm. The laminates to be tested are cut into pieces having 25 mm of width and -200 mm in machine direction. The test parameters to be adjusted in software are given in Table 4.

**Table 4**

| **Lamination Strength Test Parameters** | |
|---|---|
| **Test parameter** | **Numerical value** |
| The distance between the clamps | 25 mm |
| Test speed | 300 mm/min |
| Pre load speed | None |
| Pre load | None |
| Return speed | 300 mm/min |
| Break point | 90 % of Fmax |
| No evaluation of the test travel start | 25 mm |
| No evaluation of the test travel end | 25 mm |
| Measurement travel | 100 mm |

### Tensile Strength Test

In this test method tension strength of the base material samples of the present invention which is pretreated with atmospheric pressure plasma before hotmelt is applied on to adhere it with synthetic polymer nonwoven side panel was measured by Zwick/Roell instrument according to WSP (World Strategic Partners) 110.4 applying a pre load of 0.05 N of the samples. The laminates to be tested are cut into pieces having 50 mm of width and 250 mm.

The test parameters to be adjusted in software are given in Table 5.

**Table 5**

| **Tensile Strength Test Paramaters** | |
|---|---|
| **Test Parameter** | **Numerical Value** |
| Test speed | 1000 mm/min |
| Preload speed | 100 mm/min |
| Preload | 0,05 N |
| Wait time | 5 sec |

For performing the tests the sample is placed between the upper and lower clamps. The base sheet material of the fastening tape is the upper edge of the sample and is placed in the upper clamp and pneumatic valve connected to the upper clamp is closed to shut the clamp and nip the upper edge of the sample. The side panel sample is the lower edge of the sample and placed in the lower clamp and pneumatic valve connected to the lower clamp is closed to shut the clamp. The test is started using the instruction button on the screen of software.

The sample is pulled in the upward direction by the upper clamp of the instrument while the lower clamp is immobile. The sample is pulled until it is ruptured. After that upper clamp releases automatically and turns back to initial position. The value of maximum force for the breaking (Fₘₐₓ), and the value of elongation at break (E_{break}) appeared on the screen of the software is recorded.

## Claims

1. A method for bonding a first polyolefinic material to a polyolefinic material **characterized in that** said first and second polyolefinic materials are moving at a speed of at least 50 m/min and at least a portion of said first polyolefinic material is pretreated with an atmospheric pressure plasma while it is moving to create a pretreated portion and an adhesive is applied to at least a portion of said pretreated portion , and said pretreated portion is brought into contact with said second polyolefinic material, wherein said method is applied for preparation of pals of a disposable absorbent article selected from diaper, sanitary napkin, pull up, adult diaper, pant, pantyliner and disposable underpad.

2. A method according to claim 1, wherein said atmospheric pressure plasma is processing with air, nitrogen or argon gas, preferably with nitrogen gas.

3. A method according to claim 1, wherein said atmospheric pressure plasma is applied onto said polyolefinic material at temperatures between 160 °C to 240 °C, preferably around 200 °C and with contact temperatures between 0 °C to 50 °C, preferably between 3-25 °C.

4. A method according to claim 1, wherein said atmospheric pressure plasma is applied using single jet electrode with a distance of at least 3 mm from said polyolefinic material.

5. A method according to claim 1, wherein said polyolefinic material is a nonwoven material or a film.

6. A method according to claim 1, wherein said parts of disposable absorbent article is comprised of fastening system, waist bonding system, cuff system, landing zone, leg elastic and acquisition layer.

7. A method for bonding of a polyolefinic nonwoven material with a polyolefinic film according to claim 1, comprising the steps, (i)- treating corresponding regions of polyolefinic nonwoven material by atmospheric pressure plasma, (ii)- optionally treating corresponding regions of **polyolefinic** film by plasma, (iii)- applying adhesive or glue onto targeted regions of layer or layers, (iv)-joining said regions together.

8. A method for bonding of a fastening tape according to claim 7, comprising the steps of , (i)-treating base sheet part by atmospheric pressure plasma, (ii)-applying adhesive or glue onto nonwoven element iii) optionally treating hook element part with plasma, (iv)-joining said parts together

9. A method for bonding of a polyolefinic nonwoven material with a polyolefinic nonwoven material according to claim 1, comprising the steps, (i)-treating corresponding regions of one of the polyolefinic nonwoven material by atmospheric pressure plasma, (ii)- optionally treating regions of another polyolefinic nonwoven material by atmospheric pressure plasma, (iii)- applying adhesive or glue onto targeted regions of at least one of said polyolefinic nonwoven materials, (iv)-joining said regions together.

10. A method for bonding of a base sheet of a fastening tape to a side panel according to claim 9, comprising the steps of, (i)- treating corresponding regions of a base sheet of a fastening tape by atmospheric pressure plasma, (ii)- applying adhesive or glue onto targeted regions of at least one of said polyolefinic nonwoven materials, (iii)- attaching said treated regions of base sheet to side panel, (iv)-joining said regions together.

11. A method for bonding of polyolefinic nonwoven topsheet material of a sanitary napkin to polyolefinic film backsheet material of said sanitary napkin according to claim 1, comprising the steps, (i)- treating corresponding regions of polyolefinic nonwoven material by atmospheric pressure plasma, (ii)- optionally treating corresponding regions of polyolefinic film by plasma, (iii)- applying adhesive or glue onto targeted regions of layer or layers, (iv)-joining said regions together.

12. A method according to claim 1, wherein the amount in weight of applied adhesive in the adhesion method based on atmospheric plasma treatment is significantly less than in the adhesion method without atmospheric plasma treatment, wherein the bonding strength of atmospheric plasma-treated bonded materials and non atmospheric plasma-treated bonded materials are essentially the same.

13. A method according to claim 12, wherein the amount in weight of applied adhesive in the adhesion method based on atmospheric plasma treatment is up to 70% less than in the adhesion method without atmospheric plasma treatment, wherein the bonding strength of atmospheric plasma-treated bonded materials and non atmospheric plasma-treated bonded materials are essentially the same.

## Patentansprüche

1. Eine Methode zum Verbinden eines ersten polyolefinischen Materials, **dadurch gekennzeichnet, dass** das genannte erste und das zweite polyolefinische Materialien sich mit einer Geschwindigkeit von mindestens 50 m / min bewegen und mindestens ein Teil des genannten ersten polyolefinischen Materials während der Bewegung zum Erzeugen eines vorbehandelten Abschnitts mit einem Atmosphärendruckplasma vorbehandelt wird, und ein Klebstoff auf mindestens einen Abschnitt des genannten vorbehandelten Abschnitts aufgebracht wird, und der genannte vorbehandelte Abschnitt mit dem genannten zweiten polyolefinischen Material in Kontakt gebracht wird,
wobei die genannte Methode zur Herstellung von Teilen eines aus Windel, Damenbinde, Hochziehwindel, Windel für Erwachsene, Höschenwindel, Slipeinlage und Wegwerfunterlage ausgewählten absorbierenden Einwegartikel angewendet wird.

2. Eine Methode nach Anspruch 1, wobei das genannte Atmosphärendruckplasma mit Luft, Stickstoff oder Argongas, vorzugsweise mit Stickstoffgas, arbeitet.

3. Eine Methode nach Anspruch 1, wobei das genannte Atmosphärendruckplasma bei Temperaturen zwischen 160 ° C und 240 ° C, vorzugsweise um 200 ° C und Kontakttemperaturen zwischen 0 ° C und 50 ° C, vorzugsweise zwischen 3 und 25 ° C auf das polyolefinischen Material aufgebracht wird.

4. Eine Methode nach Anspruch 1, wobei das genannte Atmosphärendruckplasma unter Verwendung einer Einzelstrahlelektrode mit einem Abstand von mindestens 3 mm von dem polyolefinischen Material angewendet wird.

5. Eine Methode nach Anspruch 1, wobei das genannte polyolefinische Material ein Vliesstoff oder ein Film ist.

6. Eine Methode nach Anspruch 1, wobei die genannte Teile des absorbierenden Einwegartikels ein Befestigungssystem, ein Taillenbindungssystem, ein Bündchensystem, eine Landezone, ein Beinelastifizierung und eine Akqusitionsschicht umfassen.

7. Eine Methode zum Verbinden eines Polyolefinvliesmaterials mit einem Polyolefinfilm nach Anspruch 1, umfassend die Schritte (i) - Behandeln entsprechender Bereiche des Polyolefinvliesmaterials durch Atmosphärendruckplasma, (ii) - gegebenenfalls Behandeln entsprechender Bereiche des Polyolefinfilms durch Plasma, (iii) - Auftragen von Klebstoff oder Leim auf Zielregionen der Schicht oder Schichten, (iv) - Verbinden der genannten Bereiche miteinander.

8. Eine Methode zum Verbinden eines Befestigungsbandes nach Anspruch 7, umfassend die Schritte, (i)- Behandeln des Basisblattteils durch Atmosphärendruckplasma, (ii)- Auftragen von Klebstoff oder Leim auf das Viiesstoffelement (iii) - gegebenenfalls Behandeln des Hakenelementsteil mit Plasma, (iv)- Verbinden der genannten Teile miteinander.

9. Eine Methode zum Verbinden eines Polyolefinvliesmaterials mit einem Polyolefinvliesmaterial nach Anspruch 1, umfassend die Schritte (i)- Behandeln entsprechender Bereiche eines den Polyolefinvliesmaterialien durch Atmosphärendruckplasma, (ii) - gegebenenfalls Behandeln von Bereichen eines anderen Polyolefinvliesmaterials durch Atmosphärendruckplasma, (iii) - Aufbringen von Klebstoff oder Leim auf Zielregionen von mindestens einem der Polyolefinvliesmaterialien, (iv) - Verbinden der genannten Bereichen miteinander.

10. Eine Methode zum Verbinden eines Basisblatts eines Befestigungsbands mit einer Seitenfeld nach Anspruch 9, umfassend die Schritte, (i) - Behandeln entsprechender Bereiche eines Basisblatts eines Befestigungsbands durch Atmosphärendruckplasma, (ii) - Auftragen von Klebstoff oder Leim auf Zielbereiche von mindestens einen der genannten Polyolefinvliesmaterialien, (iii) - Befestigen der behandelten Bereiche des Basisblatts an dem Seitenfeld, (iv) - Verbinden der genannten Bereichen miteinander.

11. Eine Methode zum Verbinden von Polyolefinvlies-Decklagenmaterial einer Damenbinde mit Polyolefinfilm-Aussenlagematerial der Damenbinde nach Anspruch 1, umfassend die Schritte (i) - Behandeln entsprechender Bereiche von Polyolefinvliesmaterial durch Atmosphärendruckplasma, (ii) - gegebenenfalls Behandeln entsprechender Bereiche des Polyolefinfilms durch Plasma, iii) - Auftragen von Klebstoff oder Leim auf Zielbereiche der Schicht oder Schichten, (iv) - Verbinden der genannten Bereiche miteinander.

12. Eine Methode nach Anspruch 1, wobei die Gewichtsmenge an aufgetragenem Klebstoff bei dem Haftverfahren auf der Grundlage einer atmosphärischen Plasmabehandlung wesentlich geringer ist als bei dem Haftverfahren ohne atmosphärische Plasmabehandlung, wobei die Haftfestigkeit von atmosphärisch plasmabehandelten Verbundmaterialien und nicht atmosphärisch plasmabehandelten Verbundmaterialien sind in Wesentlichen das gleiche.

13. Eine Methode nach Anspruch 12, wobei die Gewichtsmenge an aufgetragenem Klebstoff bei dem Haftverfahren, bezogen auf atmosphärische Plasmabehandlung, bis zu 70% geringer ist als bei dem Haftverfahren ohne atmosphärische Plasmabehandlung, wobei die Haftfestigkeit von atmosphärisch plasmabehandelten Verbundmaterialien und nicht atmosphärisch plasmabehandelten Verbundmaterialien sind in Wesentlichen das gleiche.

## Revendications

1. Procédé pour coller un premier matériau polyoléfinique à un matériau polyoléfinique **caractérisé en ce que** lesdits premier et second matériaux polyoléfiniques se déplacent à une vitesse d'au moins 50 m/min et qu'au moins une partie dudit premier matériau polyoléfinique est prétraitée par un plasma à pression atmosphérique pendant qu'il se déplace pour créer une partie prétraitée et un adhésif est appliqué sur au moins une partie de ladite partie prétraitée, et ladite partie prétraitée est mis en contact avec ledit deuxième matériau polyoléfinique, dans lequel ledit procédé est appliqué à la préparation des parties d'un article absorbant jetable choisi parmi la couche, la serviette hygiénique, la couche-culotte, la couche pour adulte, le culotte, le protège-slip et l'alèse jetable.

2. Procédé selon la revendication 1, dans lequel ledit plasma à pression atmosphérique est traité par l'air, à l'azote ou à l'argon gazeux, de préférence par l'azote gazeux.

3. Procédé selon la revendication 1, dans lequel ledit plasma à pression atmosphérique est appliqué sur ledit matériau polyoléfinique à des températures comprises entre 160 °C et 240 °C, de préférence environ 200 °C et à des températures de contact comprises entre 0 °C et 50 °C, de préférence entre 3 °C et 25 °C.

4. Procédé selon la revendication 1, dans lequel ledit plasma à pression atmosphérique est appliqué en utilisant une unique électrode à jet avec une distance d'au moins 3 mm dudit matériau polyoléfinique.

5. Procédé selon la revendication 1, dans lequel ledit matériau polyoléfinique est un matériau non tissé ou un film.

6. Procédé selon la revendication 1, dans lequel lesdites parties de l'article absorbant jetable est constitué d'un système de fixation, d'un système d'encollage à la taille, d'un système de poignée, d'une zone d'atterrissage, des élastiques au niveau des jambes et d'une couche d'absorption.

7. Procédé pour coller un matériau non tissé polyoléfinique avec un film polyoléfinique selon la revendication 1, comprenant les étapes consistant à : (i)- traiter les régions correspondantes du matériau non tissé polyoléfinique par plasma à pression atmosphérique, (ii)- traiter, éventuellement, les régions correspondantes du film polyoléfinique par plasma, (iii)- appliquer l'adhésif ou la colle sur les régions ciblées de la couche ou des couches, (iv)- attacher lesdites régions ensemble.

8. Procédé pour coller un ruban de fixation selon la revendication 7, comprenant les étapes consistant à : (i)- traiter la partie « feuille de base » par plasma à pression atmosphérique, (ii)- appliquer l'adhésif ou la colle sur l'élément non tissé, (iii)- traiter, éventuellement, la partie « crochet » par plasma, (iv)- attacher lesdites pièces ensemble.

9. Procédé pour coller un matériau non tissé polyoléfinique avec un matériau non tissé polyoléfinique selon la revendication 1, comprenant les étapes consistant à : (i)-traiter les régions correspondantes de l'un des matériaux non tissés polyoléfiniques par plasma à pression atmosphérique, (ii)- traiter, éventuellement, des régions d'un autre matériau non tissé polyoléfinique par plasma à pression atmosphérique, (iii)-appliquer l'adhésif ou la colle sur les régions ciblées d'au moins l'un desdits matériaux non tissés polyoléfiniques; (iv)- attacher ensemble lesdites régions.

10. Procédé pour coller une feuille de base d'un ruban de fixation sur un panneau latéral selon la revendication 9, comprenant les étapes consistant à : (i)- traiter les régions correspondantes d'une feuille de base d'un ruban de fixation par plasma à pression atmosphérique, (ii)- appliquer l'adhésif ou la colle sur les régions ciblées d'au moins l'un desdits matériaux non tissés polyoléfiniques, (iii)-attacher lesdites régions traitées de la feuille de base au panneau latéral, (iv)- attacher lesdites régions ensemble.

11. Procédé pour coller le matériau de feuille supérieure non tissé polyoléfinique d'une serviette hygiénique au matériau de feuille arrière en film polyoléfinique de ladite serviette hygiénique selon la revendication 1, comprenant les étapes consistant à : (i)-traiter les régions correspondantes du matériau non tissé polyoléfinique par plasma à pression atmosphérique, (ii)-traiter, éventuellement, les régions correspondantes du film polyoléfinique par plasma, (iii)- appliquer l'adhésif ou la colle sur des zones ciblées de couche ou des couches, (iv)- attacher lesdites régions ensemble.

12. Procédé selon la revendication 1, dans lequel la quantité en poids de l'adhésif appliqué dans le procédé de collage basé sur le traitement par plasma atmosphérique est considérablement inférieure à celle du procédé de collage sans traitement par plasma atmosphérique, dans lequel force de l'adhérence des matériaux collés traités par plasma atmosphérique et des matériaux collés traités par plasma non atmosphérique sont essentiellement les mêmes.

13. Procédé selon la revendication 12, dans lequel la quantité en poids de l'adhésif appliqué dans le procédé de collage basé sur le traitement par plasma atmosphérique peut être jusqu'à 70% inférieure à celle du procédé de collage sans traitement par plasma atmosphérique, dans lequel force de l'adhérence des matériaux collés traités par plasma atmosphérique et des matériaux collés traités par plasma non atmosphérique sont essentiellement les mêmes.
